# EUROPÄISCHE PATENTSCHRIFT

(11) **EP 1 187 808 B1**
(45) Veröffentlichungstag und Bekanntmachung des Hinweises auf die Patenterteilung: **20.08.2003**
(21) Anmeldenummer: 00943828.4
(22) Anmeldetag: 16.06.2000
(51) Int. Cl.: C07C 263/10

(54) **HELLE ISOCYANATE, VERFAHREN ZU DEREN HERSTELLUNG UND DEREN VERWENDUNG**
LIGHT ISOCYANATES, METHOD FOR PRODUCING THEM AND USE THEREOF
ISOCYANATES CLAIRS, LEUR PROCEDE DE PRODUCTION ET LEUR UTILISATION

(30) Priorität: 23.06.1999 DE 19928741
(43) Veröffentlichungstag der Anmeldung: 20.03.2002
(73) Patentinhaber: BASF AKTIENGESELLSCHAFT, 67056 Ludwigshafen (DE)
(72) Erfinder: REIF, Martin, D-67063 Ludwigshafen (DE); VAN DEN ABEEL, Peter, B-2930 Brasschaat (BE); NEVEJANS, Filip, B-9120 Beveren-Waas (BE); SCHWARZ, Hans, Volkmar, B-1410 Waterloo (BE); PENZEL, Ulrich, D-01945 Tettau (DE); SCHARR, Volker, D-01968 Senftenberg (DE)
(74) Vertreter: Isenbruck, Günter, Dr.
(86) Internationale Anmeldenummer: EP0005610
(87) Internationale Veröffentlichungsnummer: WO01000569

(56) Entgegenhaltungen:
- DE-A- 4 300 774
- US-A- 3 660 261
- US-A- 5 583 251
- US-A- 5 872 278

## Beschreibung

Die Erfindung betrifft helle Isocyanate, ein Verfahren zur Herstellung von hellen Isocyanaten sowie deren Verwendung in Urethanverbindungen, insbesondere in Polyurethanen, beispielsweise in Polyurethanschäumen.

lsocyanate und Isocyanatgemische werden nach bekannten Verfahren durch Phosgenierung der entsprechenden Amine hergestellt. Für Polyurethanschäume finden beispielsweise di- oder polyfunktionelle aromatische Isocyanate der Diphenylmethandiisocyanat-Reihe (MDI) Anwendung. Bedingt durch den Herstellungsprozeß werden nach der Phosgenierung und der anschließenden Aufarbeitung (Abtrennung des Lösemittels; Abtrennen von monomerem MDI) oft dunkel gefärbte Produkte erhalten, die wiederum gelblich verfärbte Polyurethanschäume oder andere, ebenfalls verfärbte PUR-Materialien ergeben. Dies ist unerwünscht, da eine solche Färbung den visuellen Gesamteindruck beeinträchtigt und geringfügige Inhomogenitäten hervortreten läßt, z. B. als Schlieren in den erhaltenen Schäumen. Helle Isocyanate, bzw. Isocyanate, die eine reduzierte Menge an farbgebenden Komponenten enthalten, werden deshalb als Rohstoffe bevorzugt.

Es hat daher nie an Versuchen gefehlt, Polyisocyanate, insbesondere solche der Diphenylmethandiisocyanat-Reihe, mit heller Farbe zu erhalten. Zur empirischen Farbaufhellung von MDI sind zahlreiche Methoden bekannt. Die Natur der störenden Farbkörper ist bisher jedoch nur unzureichend geklärt.

Die bislang bekannten Verfahren lassen sich in vier Gruppen unterteilen:

### 1. Verfahren, bei denen das Ausgangsmaterial Diaminodiphenylmethan (MDA) bzw. dessen Oligomere einer Behandlung und/oder Reinigung unterzogen wurde

Die EP-A 0 546 398 beschreibt ein Verfahren zur Herstellung von Polymer-MDI, bei dem das als Edukt verwendete Polymethylenpolyphenylenpolyamin vor der Phosgenierung angesäuert wird.

Die EP-A 0 446 781 betrifft ein Verfahren zur Herstellung von Polymer-MDA (monomere und oligomere Polymethylenpolyphenylenpolyamine), die zuerst mit Wasserstoff behandelt und anschließend einer Phosgenierung unterzogen werden, wobei ein helleres MDI erhalten wird.

Die obengenannten Verfahren ergeben nur eine geringfügige Farbverbesserung, da die Farbkörper im MDI erfahrungsgemäß nicht nur aus bestimmten MDA-Nebenkomponenten entstehen, sondern auch aus Farbprecursoren resultieren, die durch Nebenreaktionen während der Phosgenierung gebildet werden.

### 2. Verfahrenstechnische Lösungen im Phosgenierungsprozeß

Die US-A 5 364 958 betrifft ein Verfahren zur Herstellung von Polyisocyanaten, bei dem nach der Phosgenierung das Phosgen bei niedriger Temperatur vollständig entfernt wird und anschließend das Isocyanat in der Wärme mit HCI-Gas behandelt wird.

In der DE 19817691.0 wird ein Verfahren zur Herstellung von MDI-/PMDI-Mischungen mit vermindertem Gehalt an chlorierten Nebenprodukten und verminderter Iodfarbzahl durch Einhaltung definierter Parameter in der Phosgenierungsreaktion beschrieben. Insbesondere ist hier die Einhaltung bestimmter Phosgen/HCl-Verhältnisse in der Reaktionsstufe erforderlich. Dieses Verfahren hat den Nachteil, daß eine Variation der Parameter in der Phosgenierung erschwert und dadurch die Qualität der Phosgenierung sehr anfällig wird. Durch die fehlende Flexibilität der Parameter in der Phosgenierung wird außerdem eine praktische Durchführung der Phosgenierung sehr schwierig und erfordert einen hohen technischen Aufwand.

Verfahren der genannten Art versuchen zwar an der richtigen Stelle die Verfärbungen verursachenden Komponenten abzutrennen, sie sind jedoch sowohl aufgrund ihres hohen technischen Aufwands bzw. der hohen Kosten als auch in ihrem Farbaufhellungseffekt zu wenig effizient, da der Abbau von Farbvorläufern durch unvollständig ablaufende chemische Reaktionen nur in geringem Umfang erfolgt.

### 3. Zusatz von Farbaufhellungs-Additiven zum nach der Phosgenierung und vor der Aufarbeitung erhaltenen Isocyanat-Rohprodukt.

Die EP-A 0 581 100 betrifft ein Verfahren zur Herstellung von Polyisocyanaten, bei dem nach der Phosgenierung ein chemisches Reduktionsmittel vor dem Abtrennen von Lösemittel zugegeben wird, wobei gemäß dieser Druckschrift ebenfalls helle Produkte erhalten werden.

Gemäß der US-A 4 465 639 wird dem nach der Phosgenierung erhaltenen Rohprodukt zur Farbaufhellung Wasser zugesetzt. Zum gleichen Zweck beschreiben die EP-A 538 500, EP-A 0 445 602 und EP-A 0 467 125 den Zusatz von Carbonsäuren, Alkanolen bzw. Polyetherpolyolen nach der Phosgenierung.

Die oben beschriebenen Verfahren zur Farbaufhellung sind zwar effizient, sie weisen jedoch Nachteile dahingehend auf, daß neben der Farbaufhellung die zugegebenen Additive Reaktionen mit den als Produkt anfallenden Isocyanaten eingehen und daraus beispielsweise in der Regel eine unerwünschte Verminderung des Isocyanatgehalts resultiert. Außerdem besteht die Gefahr der Bildung von unerwünschten Nebenprodukten im MDI.

### 4. Nachbehandlung des Endprodukts

Die EP-A 0 133 538 beschreibt die Reinigung von Isocyanaten durch Extraktion, wodurch Fraktionen eines hellen MDI erhalten werden.

Die EP-A 0 561 225 betrifft ein Verfahren zur Herstellung von Isocyanaten oder Isocyanatgemischen, die laut dieser Schrift keine farbgebenden Komponenten aufweisen, wobei die Isocyanate nach der Phosgenierung der entsprechenden Amine einer Wasserstoffbehandlung bei einem Druck von 1 bis 150 bar und einer Temperatur von 100 bis 180 °C unterworfen werden. Dabei werden gemäß den dort beschriebenen Beispielen Isocyanat-Endprodukte als solche oder in Form ihrer Lösungen in geeigneten Lösemitteln hydriert.

Diese farbverbessernden Nachbehandlungen der Isocyanat-Endprodukte nach der vollständigen Abtrennung des Lösemittels bei erhöhter Temperatur sind ebenfalls wenig effizient, da durch die hohen Temperaturen, die bei der Aufarbeitung, insbesondere dem Abdestillieren des Lösemittels und (bei der Herstellung von Polymer-MDI) dem Abtrennen von monomerem MDI auftreten, bereits stabile Farbkörper entstanden sind, die chemisch nur noch schwer abzubauen sind.

Der vorliegenden Erfindung liegt demgemäß die Aufgabe zugrunde, ein neues Verfahren zur Herstellung von Isocyanaten, die keine oder nur geringe Mengen an farbgebenden Komponenten aufweisen, zur Verfügung zu stellen. Eine weitere Aufgabe der Erfindung besteht darin, ein Verfahren zur Herstellung von Isocyanaten zur Verfügung stellen, das ohne die oben genannten Behandlungsschritte zu hellen Isocyanaten führt, die zur Herstellung von Polyurethanen oder deren Vorläufern geeignet sind, die keine oder nur eine geringe Färbung aufweisen.

Die erfindungsgemäße Aufgabe konnte überraschenderweise dadurch gelöst werden, daß bei der Herstellung der Isocyanate Phosgen eingesetzt wird, das weniger als 50 ppm an Brom oder bromhaltigen Verbindungen oder Iod oder iodhaltigen Verbindungen aufweist.

Gegenstand der vorliegenden Erfindung ist demnach ein Verfahren zur Herstellung von Isocyanaten durch Umsetzung eines Amins oder eines Gemischs aus zwei oder mehr Aminen mit Phosgen, dadurch gekennzeichnet, daß das eingesetzte Phosgen, weniger als 50 ppm Brom oder Iod oder deren Gemisch in molekularer oder gebundener Form enthält.

Unter Brom oder Iod in molekularer Form werden im Rahmen des vorliegenden Textes solche Moleküle verstanden, die lediglich aus Brom- oder Iodatomen bestehen. Unter Brom oder Iod in gebundener Form werden Moleküle verstanden, die neben Brom oder Iod noch weitere, von den genannten Atomen jeweils verschiedene Atome enthalten.

Das im Rahmen der vorliegenden Erfindung eingesetzte Phosgen enthält damit weniger als 50 ppm Brom oder Bromverbindungen oder Iod oder Iodverbindungen; oder Brom und Iod; oder Brom und Iod und Bromverbindungen; oder Iodverbindungen und Brom und Iod; oder Bromverbindungen und Iodverbindungen; oder Brom und Iod und Bromverbindungen und Iodverbindungen.

Das erfindungsgemäße Verfahren führt dabei zu Isocyanaten, die gewünschtenfalls ohne die oben angegebenen zusätzlichen Behandlungen bereits zur Herstellung von Urethanverbindungen wie Polyurethanen oder deren Vorläufern einsetzbar sind, die keine oder nur eine geringe Färbung aufweisen.

Das erfindungsgemäße Ergebnis war insofern besonders überraschend als bislang nicht bekannt war, daß bereits äußerst geringe Spuren von molekularem oder gebundenem Brom oder lod oder den oben genannten Gemischen im zur Herstellung von Isocyanaten eingesetzten Phosgen ausreichen, um die Produktfarbe in unerwünschter Weise beeinflussen.

Das zur Herstellung von Isocyanaten eingesetzte Phosgen weist in der Regel einen bestimmten Gehalt an molekularem oder gebundenem Brom oder Iod oder den oben genannten Gemischen auf. Der Gehalt an Brom oder Iod oder solchen Gemischen im Phosgen resultiert aus dem zur Phosgenherstellung eingesetzten Chlor, das üblicherweise einen bestimmten Anteil an Brom oder Iod oder deren Gemisch aufweist. Der Gehalt des Chlors an Brom oder Iod oder deren Gemisch resultiert wiederum in der Regel aus dem entsprechenden Gehalt des zur Chlorherstellung verwendeten Salzes. Bislang war jedoch anzunehmen, daß das im Chlor bei der Phosgensynthese vorliegende Brom bzw. BrCl zur Bildung von Dibromphosgen bzw. Brom-Chlorphosgen (analog zur Bildung von COBrF aus CO + BrF₃; s. W. Kwasnik in "Handbuch der präparativen anorganischen Chemie", Herausgeber: G. Brauer, Band 1, 3. Aufl., Ferdinand Enke Verlag, Stuttgart, 1975, S. 224) führt. Diese Verbindungen sollten dann, analog zum Phosgen, mit Aminen unter Bildung von Isocyanaten und Bromwasserstoff reagieren (US 2 733 254). Analoge Reaktionen sind für Iod anzunehmen.

Das im Rahmen der vorliegenden Erfindung einzusetzende Phosgen mit niedrigem Gehalt an Brom oder Iod oder den oben genannten Gemischen kann auf verschiedene, dem Fachmann bekannte Arten hergestellt werden. Eine Möglichkeit einen niedrigen Gehalt an Brom oder Iod oder den oben genannten Gemischen im Phosgen zu garantieren ist beispielsweise die Verwendung von Ausgangsverbindungen bei der Phosgenherstellung, die bereits einen entsprechend niedrigen Gehalt an Brom oder Iod oder deren Gemisch aufweisen. Insbesondere bietet sich hierbei die Verwendung von Chlor mit einem entsprechend niedrigen Gehalt an Brom oder Iod oder deren Gemisch an.

Verfahren zur Herstellung von entsprechendem Chlor mit einem niedrigen Gehalt an Brom oder Iod oder deren Gemisch sind in der Fachwelt bekannt. Grundsätzlich kann im Rahmen der vorliegenden Erfindung jedes Chlor eingesetzt werden, das die o. g. Spezifikation erfüllt, d.h., weniger als etwa 50 ppm Brom oder Iod oder den oben genannten Gemischen, beispielsweise 25 ppm oder weniger, aufweist. So kann beispielsweise Chlor eingesetzt werden, das durch ElektrolyseVerfahren oder Oxidation von HCl, z.B. nach dem Deacon-Prozeß hergestellt wurde. In der US 3,660,261 wird die Herstellung von Chlor mit einem besonders niedrigen Gehalt an Brom durch oxidative Behandlung des für die Elektrolyse verwendeten Salzes beschrieben. Alternativ dazu ist die Entfernung von Brom oder Iod oder deren Gemisch aus Chlor mittels Destillation, selektiver Kondensation des Broms oder Iods im Chlorstrom oder durch Reaktionen mit Stoffen, die selektiv mit Brom oder Iod oder deren Gemisch reagieren, möglich, wie es beispielsweise in der JP 0075319 beschrieben wird. Natürlich können auch entsprechend geeignete Ausgangsstoffe für die Chlorsynthese eingesetzt werden, die selbst im wesentlichen kein Brom oder Iod oder deren Gemisch enthalten, z. B. im wesentlichen brom- und iodfreies Salz oder brom- und iodfreie HCl. Geeignete Verfahren sind beispielsweise in der DE-OS 18 00 844, der DE-AS 12 55 643 oder in der DE-A1 197 26 530 beschrieben.

Eine weitere Möglichkeit zur Herstellung von bromarmem Chlor ist in Ullmann's Encyclopedia of Industrial Chemistry, 5. Auflage, Band A6, S. 463 und Abbildung 70 auf S. 465 beschrieben: Bei der Wäsche von gasförmigem bromreichem Chlor mit flüssigem bromarmen Chlor im Gegenstrom verarmt das zu reinigende Chlorgas an Brom und das flüssige Chlor reichert sich mit Brom an. Zur Inbetriebnahme einer entsprechenden Anlage ist die Bereitstellung einer ausreichenden Menge an bromarmen flüssigen Chlor notwendig, anschließend kann ein Teilstrom des gewonnenen bromärmeren Chlors verflüssigt und zur Wäsche des bromreicheren Chlors benutzt werden. Dieses Verfahren wird in Kolonnen mit üblichen trennwirksamen Einbauten wie Böden, Füllkörpern oder Packungen durchgeführt. Der erreichte Abreicherungsgrad an Brom oder Iod oder deren Gemisch hängt in der für Absorptions- und Destillationsverfahren üblichen Weise von Systemdruck, den Strommengen, den Konzentrationen und den verwendeten Einbauten ab; die Auslegung der Kolonne anhand des gewünschten Brom-Abreicherungsgrads ist daher eine reine Routineaufgabe.

Das so erhältliche, weitgehend brom- und iodfreie Chlor kann anschließend im Rahmen üblicher und bekannter Prozesse wie sie z. B. in Ullmanns Enzyklopädie der industriellen Chemie, 3. Aufl., Bd. 13, Seite 494-500 beschrieben sind, zu Phosgen umgesetzt werden.

Ein weiterer Weg Phosgen mit einem niedrigen Gehalt an molekularem oder gebundenem Brom oder Iod oder deren Gemisch zu erhalten ist die Abtrennung von molekularem und gebundenem Brom und Iod aus dem Phosgen selbst. Auch hier können im Prinzip wieder alle gängigen Trennverfahren eingesetzt werden, beispielsweise Destillation, Adsorption und dergleichen. Für das erfindungsgemäße Verfahren ist letztlich ausschließlich die Einhaltung der oben genannten Obergrenze für die Konzentration an molekularem oder gebundenem Brom oder Iod oder den oben genannten Gemischen entscheidend.

In einer bevorzugten Ausführungsform der Erfindung wird Phosgen eingesetzt, das einen Gehalt an Brom oder Iod oder den oben genannten Gemischen von weniger als 40 ppm, 35 ppm, 30 ppm oder 25 ppm oder weniger, insbesondere von 10 ppm oder weniger aufweist.

Die im Rahmen des erfindungsgemäßen Verfahrens stattfindende Isocyanatherstellung wird in einer dem Fachmann bekannten Weise durch Umsetzung eines Amins oder eines Gemischs aus zwei oder mehr Aminen mit Phosgen in überstöchiometrischer Menge durchgeführt. Anwendbar sind grundsätzlich alle Verfahren, bei denen ein primäres Amin oder ein Gemisch aus zwei oder mehr primären Aminen mit Phosgen unter Bildung einer oder mehrerer Isocyanatgruppen umgesetzt wird.

In einer bevorzugten Ausführungsform der Erfindung wird das erfindungsgemäße Verfahren, d.h., die Umsetzung des Amins oder des Gemischs aus zwei oder mehr Aminen mit dem Phosgen in einem Lösemittel oder einem Gemisch aus zwei oder mehr Lösemitteln durchgeführt.

Als Lösemittel können alle für die Herstellung von Isocyanaten geeigneten Lösemittel eingesetzt werden. Vorzugsweise sind dies inerte aromatische, aliphatische oder alicyclische Kohlenwasserstoffe bzw. deren halogenierte Derivate. Beispiele für solche Lösemittel sind aromatische Verbindungen wie Mono- oder Dichlorbenzol, beispielsweise o-Dichlorbenzol, Toluol, Xylole, Naphthalinderivate wie Tetralin oder Decalin, Alkane mit etwa 5 bis etwa 12 C-Atomen wie Hexan, Heptan, Octan, Nonan oder Decan, Cycloalkane wie Cyclohexan, inerte Ester und inerte Ether wie Ethyl- oder Butylacetat, Tetrahydrofuran, Dioxan oder Diphenylether.

Als Amine eignen sich prinzipiell alle primären Amine die in geeigneter Weise mit Phosgen zu Isocyanaten reagieren können. Geeignet sind prinzipiell alle linearen oder verzweigten, gesättigten oder ungesättigten aliphatischen oder cycloaliphatischen oder aromatischen primären Mono- oder Polyamine, sofern diese mit Phosgen zu Isocyanaten umgesetzt werden können. Beispiele für geeignete Amine sind 1,3-Propylendiamin, 1,4-Butylendiamin, 1,5-Pentamethylendiamin, 1,6-Hexamethylendiamin und die entsprechenden höheren Homologen dieser Reihe, Isophorondiamin (IPDA), Cyclohexyldiamin, Cyclohexylamin, Anilin, Phenylendiamin, p-Toluidin, 1,5-Naphtylendiamin, 2,4- oder 2,6-Toluylendiamin oder deren Gemisch, 4,4'-, 2,4'- oder 2,2'-Diphenylmethandiamin oder deren Gemische sowie höhermolekulare isomere, oligomere oder polymere Derivate der obengenannten Amine und Polyamine. Im Rahmen einer bevorzugten Ausführungsform der vorliegenden Erfindung wird als Amin ein Amin der Diphenylmethandiamin-Reihe oder ein Gemisch aus zwei oder mehr solcher Amine eingesetzt.

Nach Durchlaufen des erfindungsgemäßen Verfahrens liegen die obengenannten Verbindungen in Form der entsprechenden Isocyanate vor, z.B. als 1,6-Hexamethylendiisocyanat, Isophorondiisocyanat, Cyclohexylisocyanat, Cyclohexyldiisocyanat, Phenylisocyanat, Phenylendiisocyanat, 4-Toluylisocyanat, 1,5-Naphthylendiisocyanat, 2,4- oder 2,6-Toluylendiisocyanat oder deren entsprechende Gemische, 4,4'-, 2,4'- oder 2,2'-Diphenylmethandiisocyanat oder Gemische aus zwei oder mehr davon, sowie als höhermolekulare oligomere oder polymere Derivate der obengenannten Isocyanate oder als Gemische aus zwei oder mehr der genannten Isocyanate oder Isocyanatgemische.

In einer bevorzugten Ausführungsform der vorliegenden Erfindung werden als Amine die isomeren, primären Diphenylmethandiamine (MDA) bzw. deren oligomere oder polymere Derivate eingesetzt, d.h., die Amine der Diphenylmethandiamin-Reihe. Diphenylmethandiamin, dessen Oligomere oder Polymere werden beispielsweise durch Kondensation von Anilin mit Formaldehyd erhalten. Auch solche Oligo- oder Polyamine oder deren Gemische werden im Rahmen einer bevorzugten Ausführungsform der Erfindung eingesetzt.

Die Umsetzung des im Rahmen der vorliegenden Erfindung einzusetzenden und im Rahmen der oben genannten Einschränkungen brom- und iodarmen oder sogar brom- und iodfreien Phosgens mit einem der obengenannten Amine oder einem Gemisch aus zwei oder mehr solcher Amine, kann kontinuierlich oder diskontinuierlich in einer oder mehreren Stufen erfolgen. Wird eine einstufige Umsetzung durchgeführt, so erfolgt diese Umsetzung vorzugsweise bei etwa 60 bis 200 °C, beispielsweise bei etwa 130 bis 180 °C.

In einer weiteren Ausführungsform der Erfindung kann die Umsetzung beispielsweise auch zweistufig durchgeführt werden. Hierbei wird in einer ersten Stufe die Umsetzung des Phosgens mit dem Amin oder dem Gemisch aus zwei oder mehr Aminen, bei einer Temperatur zwischen etwa 0 und etwa 130 °C, beispielsweise etwa 20 bis etwa 110 °C oder etwa 40 bis etwa 70 °C durchgeführt, wobei für die Reaktion zwischen Amin und Phosgen eine Zeitspanne von etwa 1 min bis etwa 2 h eingeräumt wird. Anschließend wird in einer zweiten Stufe die Temperatur, beispielsweise während einer Zeitspanne von etwa 1 min bis etwa 5 h, beispielsweise innerhalb von etwa 1 min bis etwa 3 h, auf etwa 60 bis etwa 190 °C, insbesondere etwa 70 bis 170 °C, erhöht.

In einer bevorzugten Ausführungsform der Erfindung wird die Umsetzung in zwei Stufen durchgeführt.

Während der Umsetzung kann in einer weiteren bevorzugten Ausführungsform der Erfindung erhöhter Druck angelegt werden, beispielsweise bis zu etwa 100 bar oder weniger, beispielsweise etwa 1 bar bis etwa 50 bar oder etwa 2 bar bis etwa 25 bar oder etwa 3 bar bis etwa 12 bar. Die Umsetzung kann jedoch auch drucklos erfolgen.

In einer weiteren bevorzugten Ausführungsform der Erfindung wird daher bei Umgebungsdruck, in der Regel etwa 1 bar gearbeitet. In einer weiteren bevorzugten Ausführungsform kann zudem bei gegenüber dem Umgebungsdruck reduziertem Druck gearbeitet werden.

Überschüssiges Phosgen wird vorzugsweise im Anschluß an die Umsetzung bei einer Temperatur von etwa 50 bis 180 °C entfernt. Die Entfernung der restlichen Spuren des Lösemittels erfolgt vorzugsweise unter vermindertem Druck, beispielsweise sollte der Druck etwa 500 mbar oder weniger, bevorzugt weniger als 100 mbar betragen. Im Allgemeinen werden dabei die verschiedenen Komponenten in der Reihe der Siedepunkte abgetrennt, wobei auch die Abtrennung von Gemischen der verschiedenen Komponenten in einer einzigen Verfahrensstufe möglich ist.

Ein weiterer Gegenstand der Erfindung sind helle Isocyanate, wie sie nach dem erfindungsgemäßen Verfahren herstellbar sind.

Ein weiterer Gegenstand der Erfindung ist die Verwendung von Isocyanaten, herstellbar gemäß dem erfindungsgemäßen Verfahren oder nach einem solchen Verfahren hergestellt, zur Herstellung von Urethanverbindungen, insbesondere von Polyurethanen. Im Rahmen einer bevorzugten Ausführungsform der Erfindung werden die erfindungsgemäßen Isocyanate bei der Herstellung von Polyurethanschäumen eingesetzt, wie sie beispielsweise als Hartschäume, Halbhart-, Integralund Weichschäume im Handel erhältlich sind.

Die Erfindung wird nachfolgend durch Beispiele näher erläutert.

Bei allen Beispielen wurde der Bromgehalt im Phosgen aus dem Bromgehalt des für die Phosgensynthese verwendeten Chlors errechnet. Der Bromgehalt im Chlor wurde mittels Röntgenfluoreszenzanalyse bestimmt.

Die in den Beispielen enthaltenen Viskositätsangaben wurden auf einem Lauda CD 20 Viskosimeter bei 25°C erhalten.

### Beispiel 1: Herstellung der MDI-Proben:

100 g Polymer-MDA, gelöst in 1,3 Liter Monochlorbenzol werden in einem 6 Liter Rührreaktor mit 200 g Phosgen, gelöst in 1,3 Liter Monochlorbenzol, bei 50 bis 80 °C, drucklos umgesetzt. Die Temperatur wird während 1 - 2 Stunden auf etwa 120 °C erhöht, wobei die Umsetzung zu den Isocyanaten (125g) erfolgt. Anschließend wird unter schonenden Bedingungen (110 °C, 100 mbar) Restphosgen und Monochlorbenzol abdestilliert. Die lösemittelfreie Roh-MDI Probe wird anschließend 45 Minuten bei 180 °C und einem Druck von 10 mbar nachbehandelt.

Die Phosgenierungen gemäß den Beispielen 1 bis 3 wurden unter konstanten Bedingungen durchgeführt. Die Versuche unterschieden sich lediglich durch den Bromgehalt im Phosgen.

### Beispiel 1:

a. Für die Synthese wurde Phosgen mit einem Bromgehalt von weniger als 10 ppm eingesetzt.
b. Für die Synthese wurde Phosgen mit einem Bromgehalt von 50 ppm eingesetzt.
c. Für die Synthese wurde Phosgen mit einem Bromgehalt von 100 ppm eingesetzt.

Die Kenndaten der Produkte sind in Tabelle 1 angeführt.

### Kenndaten der Endprodukte:

Von den als Beispiele 1 bis 3 hergestellten Produkten wurden die Isocyanat-Kenndaten bestimmt. Es wurde speziell die für MDI üblicherweise angegebene Iod-Farbzahl bestimmt. Hierzu wurden die Proben (1:5 verdünnt in Monochlorbenzol) mit einem Photometer (Firma Dr. Lange, Berlin) im IFZ-Programm-Modus gemessen.

**Tabelle 1:**

| Kenndaten der Beispiele | | | |
|---|---|---|---|
| | Bromgehalt im Phosgen (ppm) | NCO¹(%) | IFZ² |
| Beispiel 1 | < 10 | 32,2 | 18,9 |
| Beispiel 2 | 50 | 32,2 | 24,1 |
| Beispiel 3 | 100 | 32,3 | 28,6 |

| | | | |
|---|---|---|---|
| ¹ = NCO-Gehalt (ermittelt nach ASTM D 5155) | | | |
| ² = Iod-Farbzahl | | | |

Die Ergebnisse zeigen eine, gute Farbaufhellung von Roh-MDI bei Verwendung von bromarmem Chlor.

### Beispiel 2:

In einem technischen Verfahren wurden 7,9 t/h Roh-MDA mit 20,6 t/h Phosgen in Chlorbenzol als Prozeßlösemittel bei 95 °C in einer Rührkesselkaskade zum Isocyanat umgesetzt. Das die Phosgenierung verlassende Gemisch wurde entsprechend dem Stand der Technik von Phosgen und Chlorbenzol befreit und thermisch nachbehandelt. Anschließend wurde von dem so erhaltenen Roh-MDI noch ein Teil Monomer-MDI abgetrennt, so daß ein Polymer-MDI mit einer Viskosität von ca. 200 mPas erhalten wurde. Von diesem Produkt wurden die Isocyanat-Kenndaten bestimmt (Tabelle 2).

**Tabelle 2:**

| Isocyanat-Kenndaten des Beispiels 2 | | | |
|---|---|---|---|
| | Bromgehalt im Phosgen (ppm) | NCO (%) | IFZ |
| Beispiel 2a | 40 | 31,5 | 15 |
| Beispiel 2b | 20 | 31,5 | 12 |
| Beispiel 2c | 10 | 31,5 | 10 |

### Beispiels 3:

In einem technischen Verfahren wurden 3,3 t/h Roh-MDA mit 9,2 t/h Phosgen in Chlorbenzol als Prozeßlösemittel in einer Ventilbodenkolonne bei 110 °C Sumpftemperatur zum Isocyanat umgesetzt. Anschließend wurden in einer Abfolge mehrerer Destillationskolonnen überschüssiges Phosgen und Prozeßlösemittel bei Temperaturen von 130 bis 180 °C abdestilliert. Von diesem Produkt wurden die Isocyanat-Kenndaten bestimmt (Tabelle 3).

**Tabelle 3:**

| Isocyanat-Kenndaten des Beispiels 3 | | | |
|---|---|---|---|
| | Bromgehalt im Phosgen (ppm) | NCO (%) | IFZ |
| Beispiel 3a | 120 | 31,7 | 26 |
| Beispiel 3b | 70 | 31,7 | 18 |
| Beispiel 3c | 30 | 31,6 | 13 |

## Patentansprüche

1. Verfahren zur Herstellung von Isocyanaten durch Umsetzung eines Amins oder eines Gemischs aus zwei oder mehr Aminen mit Phosgen, **dadurch gekennzeichnet, daß** das eingesetzte Phosgen, weniger als 50 ppm Brom oder Iod oder deren Gemisch in molekularer oder gebundener Form enthält.

2. Verfahren nach Anspruch 1, **dadurch gekennzeichnet, daß** das Phosgen weniger als 25 ppm Brom oder Iod oder deren Gemisch in molekularer oder gebundener Form enthält.

3. Verfahren nach Anspruch 1 oder 2, **dadurch gekennzeichnet, daß** die Umsetzung in einem Lösemittel durchgeführt wird.

4. Verfahren nach einem der Ansprüche 1 bis 3, **dadurch gekennzeichnet, daß** als Amin ein Amin der Diphenylmethandiamin-Reihe oder ein Gemisch aus zwei oder mehr solcher Amine eingesetzt wird.

5. Verfahren nach einem der Ansprüche 1 bis 4, **dadurch gekennzeichnet, daß** es in einer oder zwei Stufen durchgeführt wird.

6. Verfahren nach einem der Ansprüche 1 bis 5, **dadurch gekennzeichnet, daß** die Umsetzung unter erhöhtem Druck oder drucklos durchgeführt wird.

## Claims

1. A process for preparing isocyanates by reacting an amine or a mixture of two or more amines with phosgene containing less than 50 ppm of bromine or iodine or their mixtures in molecular or bound form.

2. A process as claimed in claim 1, wherein the phosgene contains less than 25 ppm of bromine or iodine or mixtures thereof in molecular or bound form.

3. A process as claimed in claim 1 or 2, wherein the reaction is carried out in a solvent.

4. A process as claimed in any of claims 1 to 3, wherein the amine used is an amine of the diphenylmethanediamine series or a mixture of two or more such amines.

5. A process as claimed in any of claims 1 to 4 carried out in one or two stages.

6. A process as claimed in any of claims 1 to 5, wherein the reaction is carried out under superatmospheric pressure or atmospheric pressure.

## Revendications

1. Procédé pour la préparation d'isocyanates par la mise en réaction d'une amine ou d'un mélange de deux amines ou plus avec du phosgène, **caractérisé en ce que** le phosgène mis en oeuvre contient moins de 50 ppm de brome ou d'iode ou deleur mélange sous forme moléculaire ou liée.

2. Procédé selon la revendication 1, **caractérisé en ce que** le phosgène contient moins de 25 ppm de brome ou d'iode ou de leur mélange sous forme moléculaire ou liée.

3. Procédé selon la revendication 1 ou 2, **caractérisé en ce qu'**on effectue la mise en réaction dans un solvant.

4. Procédé selon l'une quelconque des revendications 1 à 3, **caractérisé en ce qu'**on met en oeuvre, à titre d'aminé, une amine de la série de la diphénylméthanediamine ou encore un mélange de deux amines de ce type ou plus.

5. Procédé selon l'une quelconque des revendications 1 à 4, **caractérisé en ce qu'**on le met en oeuvre en une ou en deux étapes.

6. Procédé selon l'une quelconque des revendications 1 à 5, **caractérisé en ce qu'**on effectue la mise en réaction sous pression augmentée ou en l'absence de pression.
